# EUROPEAN PATENT APPLICATION

(11) **EP 3 957 334 A1**
(43) Date of publication of application: **23.02.2022**
(21) Application number: 20192037.8
(22) Date of filing: 20.08.2020
(51) Int. Cl.: A61L 2/10, A61L 9/12, A61L 9/20, A61L 9/22, B60H 3/00, F24F 8/22

(54) **MOBILE AIR PURIFYING DEVICE**

(71) Applicant: PMI Labortechnik GmbH, 8114 Dänikon (CH)
(72) Inventor: HALTINER, Claude, 5430 Wettingen (CH)
(74) Representative: Toleti, Martin

(57) **Abstract**

A mobile air purifying device (1) comprises a housing (2) with an inlet (21) and an outlet (22) for air and an air path (23) through the housing (2) between the inlet (21) and the outlet (22). Further, the mobile device (1) comprises a fan (3) adapted to draw air along the air path (23) through the housing (2), a UV-C radiation source (4) adapted to emit UV-C radiation onto a part of the air path (23) and a source of negative ions (5) adapted to generate ions in the air path (23).

## Description

### Technical Field

The invention relates to a mobile device for purifying air, in particular for disinfecting air in an interior room or in a passenger compartment.

### Background Art

Various devices for purifying air are known, including mechanical filters, air ionizers for eliminating airborne bacterial and viral infections, and ultraviolet (UV) germicidal irradiation. In some devices, different of these purifying methods are combined. The devices are useful for cleaning air in an interior room, e.g. in the food industry or in a lab environment where dust and/or bacteria are undesired and detrimental to manufactured or analysed samples.

Conventional air purifying devices are, however, stationary systems, i.e. they are placed in one location, e.g. in the lab room, and hard to move afterwards. This also results from their large dimensions, e.g. of larger than 1 m x 0.5 m x 0.5 m, and their heavy weight, e.g. above 30 kg. This makes conventional air purifying devices inflexible in their use in varying settings such as e.g. in a meeting room with a changing number of persons or in a passenger compartment of a car. They cannot be set up at the expected hot spot of bacterial of viral contamination. The reduces the purifying effect.

Especially in view of airborne transmitted diseases, e.g. via bacteria or viruses in aerosols, it is desirable to provide an air purifying device which is flexible in use and can be optimally adjusted to different settings in indoor environments.

### Disclosure of the Invention

The problem to be solved by the present invention is therefore to provide a device for purifying air which is flexible in use and can easily be adjusted to varying settings in interior rooms such as in a meeting room or in a passenger compartment of a car. "Purifying" air in that respect means removing contaminants, e.g. bacteria or viruses, from the air in a room, in particular in order to improve indoor air quality and prevent allergies and diseases. "Purifying" air may also encompass mechanically removing particles such as fine dust from the air.

This problem is solved by a mobile air purifying device which comprises
- a housing with an inlet and an outlet for air: The housing may be made from a polymer, or from a metal such as aluminium, from a combination or both. In embodiments, the housing may essentially have the shape of a cylinder, a cuboid or a cone. Inlet and/or outlet may be embodied as either one opening or several neighbouring openings or slits.
- an air path through the housing between the inlet and the outlet: The air path is the path that air from a surroundings of the device entering the device is intended to travel. Typically, the air path is formed by pipes and/or channels.
- a fan adapted to draw air along the air path through the housing: The fan may comprise an electric motor and fan blades. In different embodiments, the fan may be located nearer to the inlet than to the outlet, thus pushing the air through along the air path; or the fan may be located nearer to the outlet than to the inlet, thus rather pulling the air along the air path.
- a UV-C radiation source adapted to emit UV-C radiation onto a part of the air path: In particular, the UV-C radiation source comprises a UV-C LED, which has the advantage of having a small form factor and of consuming little power. UV-C radiation, i.e. electromagnetic radiation in a frequency band between 100 and 280 nm, may be used for purifying air. The purifying mechanism of UV is a photochemical process. Contaminants in the indoor environment are almost entirely organic carbon-based compounds, which break down when exposed to high-intensity UV-C, in particular in the frequency band between 240 to 280 nm. Such ultraviolet radiation can destroy DNA in living microorganisms and RNA of viruses.
- a source of negative ions adapted to generate negative ions in the air path: In particular, the source of negative ions comprises a negative ion generator adapted to ionize air molecules, in particular at least 5 million, more particularly at least 8 million, air molecules per second. Typically, the source of negative ions applies a high voltage, e.g. above 1000 V, to ionise, i.e. electrically negative charge, air molecules, thus generating e.g. N₂⁻ and O₂⁻ molecules. In an embodiment, the negative ions are exhausted through the outlet and then electrostatically attract and bind to particles, microorganisms and viruses in the air, forming a cluster which due to its heavier weight sinks to ground. Thus, the cluster is removed from the air. The cluster may either stay on the ground, leading to a dehydration of the cluster and thus elimination of the contaminants, or the cluster may be drawn into the device where any contaminants may be eliminated, e.g. through the UV-C radiation source.

Evidently, a combination of the UV-C radiation source and the source of negative ions in one device is an efficient means to purify and in particular disinfect the air. In this respect, it is preferred that the air path is vertically oriented in the device, in particular wherein the vertical dimension of the device exceeds the two horizontal dimensions length and width, such that clusters drop to ground in the vicinity of the device which facilitates the cluster being drawn into the device again for eliminating any contamination.

The device may also comprise further purifying means, examples of which are detailed further below.

### Mobile device

The air purifying device is a "mobile" device, meaning that it is not a stationary device but a portable device. This is reflected in the following advantageous features of embodiments of the device: In an embodiment, the device weighs less than 1 kg, in particular less than 0.5 kg. Alternatively or additionally, the housing may have a height of less than 30 cm, a width of less than 10 cm and a length of less than 10 cm. Such dimensions and weight make the device portable such that it can easily be adjusted to varying settings, e.g. by putting the device in a corner or on a desk in a meeting room, or by transferring the device into the passenger compartment of a car. Such flexible location options are particularly advantageous in that the device may be placed where the air purifying effect is actually desired, e.g. in the vicinity of people, in order to prevent the transmission of diseases via airborne bacteria or viruses. Hence, the mobile device can be placed at the hotspot of anticipated virus or bacteria exposure, which, for example, in a meeting room may be on top of in the middle of the desk. Stationary device cannot achieve this; rather, stationary devices are place remote in a room, e.g. behind a door, in order not to disturb activities in the room. However, such places are typically not hotspots for the concentration of viruses or bacteria which are rather places in the vicinity of humans, for example. Hence, a light-weight, portable device of the above dimensions can simply be put in the middle of a meeting table, equivalent to a Bluetooth speaker, or a conference telephone, and be present and ready for decontamination of viruses and bacteria ejected by humans when talking or coughing.

The "mobile" aspect of the device may further be supported by the following features: The device advantageously comprises a battery for supplying power to the fan, the UV-C radiation source and the source of ions, independent from a location of the device. In particular, the battery may be adapted to be recharged via a USB port of the device. In this way, the device is adapted for use e.g. in a car having a power supply via USB. Further the battery may be adapted to supply power to an external device via the USB port. These features, again, make the device handy and flexible in use.

In spite of its small form factor and of being battery-powered, the device may still achieve a coverage, i.e. an area of sufficient elimination of contaminants, of 6 m² or more, or preferably even 10 m² or more. This makes the mobile device suitable for most meeting rooms and in particular for passenger compartments in cars.

### Further advantageous features

The device may further comprise a bottom support for supporting the housing in regular operation. In that case, the inlet may be arranged closer to the bottom support than the outlet. In conjunction with the source of negative ions, such arrangement of the inlet and the outlet supports the air purifying effect of the device. This is due to the fact that the heavy clusters formed from contaminants and negative ions in the air sink to the bottom and may then be drawn into the air path through the inlet. For that purpose the inlet may in particular be arranged in a side wall of the housing adjacent to the bottom support.

On the other hand, ions generated by the source of ions are advantageously exhausted from the air path to the top in order to be distributed over a larger area of the surroundings. Therefore, the outlet is advantageously arranged in an upper wall of the housing opposite to the bottom support.

In an advantageous embodiment, the device further comprises a mechanical air filter within the air path. Such filter mechanically removes particles and/or contaminants of a certain size from the air drawn through the filter.

In particular, the mechanical air filter may comprise at least one of the following:
- a dust filter: The dust filter may comprise a mesh to filter out particles that are e.g. larger than 10 µm from the air. Such dust filter is particularly suited as first filter stage in order to remove coarse particles from the air, thus preventing clogging of subsequent filter stages.
- an activated carbon filter: The activated carbon filter typically comprises a porous material that can adsorb volatile chemicals on a molecular basis. Thus, the activated carbon filter is in particular adapted to neutralize gases and odours in the air.
- a high-efficiency particulate air (HEPA) filter, in particular of HEPA class H13 or higher purifying efficiency. The HEPA filter may have a mesh size of e.g. as small as 0.3 µm in diameter, thus removing fine particles such as smoke, pollen, bacteria and a majority of viruses from the air.

The mechanical air filters, if any, are advantageously arranged in the air path closer to the inlet than the UV-C radiation source and the source of negative ions.

Further, the device may comprise an odour source, in particular a pad for receiving scented oil, adapted to odorize air drawn through the air path. Examples for scented oil are lavender or eucalyptus oil. Such odour source may support to create an agreeable room scent and atmosphere. Moreover, the scented oil may have an anti-bacterial effect. While the odour source may be comprised in the device, it does not always have to be applied by the user. Rather, the odour source may be switched on or off, e.g. by applying the scented oil to the pad or not. Advantageously, such odour source is located in the air path close to the outlet, in particular after the mechanical air filters and the UV-C radiation source in direction of intended air travel. A reservoir for scented oil may be arranged in a bottom part of the device.

In a further embodiment, the device comprises
- an air quality sensor, in particular a PM2.5 sensor, adapted to sense a quality of air drawn into the inlet. PM2.5, i.e. a concentration of particles with diameters of 2.5 µm and smaller, may be used as an indicator for air quality. Advantageously, the air quality sensor is located in the air path close to the inlet, in particular before the first filter stage or the UV-C radiation source in direction of intended air travel.
- a visual output unit connected to the air quality sensor and adapted to visually output an air quality value from the air quality sensor. In particular, the visual output unit may comprise a multicolor LED or at least two LEDs of different colors for a color-coded display of the air quality, e.g. red indicating "bad" air quality, i.e. high PM2.5 concentration, and green indicating "good" air quality, i.e. low PM2.5 concentration. Or, in a four-categories color-coding, red may again indicate "bad" air quality, and orange, yellow and blue may indicate gradually "better" air quality.

In yet another embodiment, the device comprises an input unit and a control unit adapted to control the fan, the UV-C radiation source and the source of ions according to a user input to the input unit. In this way, different operation parameters of the device may be controllable by the use, e.g. an air throughput per minute, a UV-C radiation intensity or a repetition frequency of UV-C flashes, and/or a rate of ions generated per second. The input unit may be embodied as a switch.

In an advantageous embodiment, the input unit may comprise a gesture sensor. In particular, the gesture sensor may comprise an IR transmitter and receiver, adapted to contactlessly sense a user input. In this way, the user may control the device without touching it. Such contactless operation of the device improves hygienic conditions and prevents a transmission of diseases.

According to another aspect of the present invention, a method is provided for purifying air, preferably by means of a mobile air purifying device according to any of the preceding embodiments. Negative ions generated by the negative ion source are exhausted or ejected through the outlet into the ambient air for the negative ions electrostatically binding to one or more of particles, microorganisms or viruses in the ambient air, resulting in a cluster sinking to ground. Such cluster is drawn, by means of the fan, via the inlet into the air path of the device. In case the cluster is of a certain size, e.g. 0.3 µm or more, which may include several molecules contributing to the cluster, the cluster may be removed from passing the air path by the HEPA filter due to its smaller mesh size. Accordingly, such clusters may be collected by the filter and hence, removed from the ambient air. In case the cluster is of smaller size, and e.g. may only hold a negative ion and a virus, for example, it may pass the HEPA filter and any of the other filters. However, the this cluster becomes irradiated by UV-C radiation emitted by the UV-C radiation source for destroying or inactivating any of the particles, microorganisms or viruses bound in the cluster.

Other advantageous embodiments are listed in the dependent claims as well as in the description below.

### Brief Description of the Drawings

The invention will be better understood and objects other than those set forth above will become apparent from the following detailed description thereof. Such description makes reference to the annexed drawings, wherein:
Fig. 1 shows a perspective view of a mobile air purifying device according to an embodiment of the invention;
Fig. 2 shows a block diagram of functional elements of a mobile air purifying device according to an embodiment of the invention.

### Modes for Carrying Out the Invention

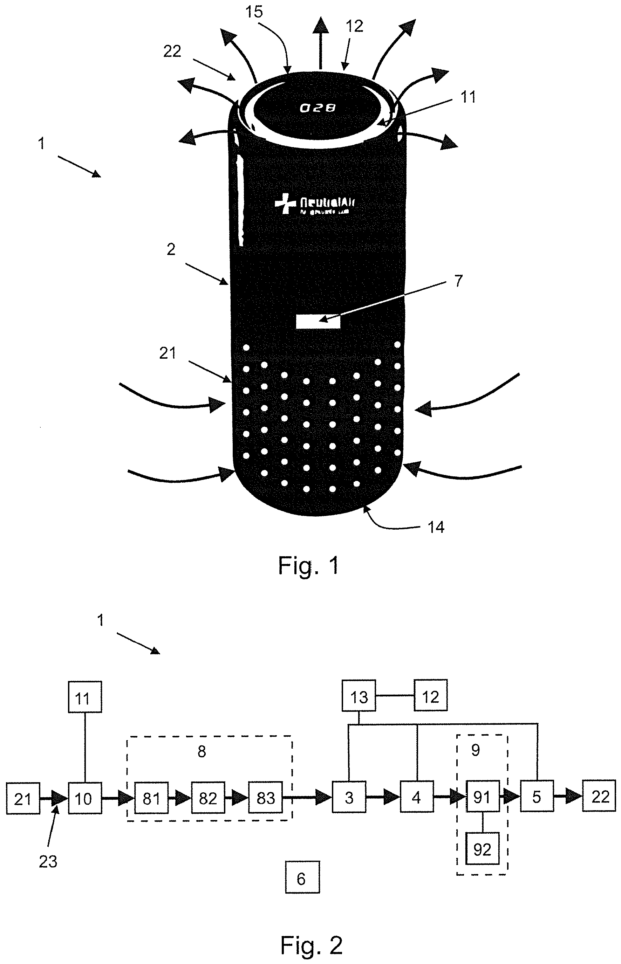

The mobile air purifying device 1 of Fig. 1 comprises a housing 2, which has an essentially cylindrical shape that gives the device 1 an appealing look. In general, other shapes of the housing 2 are possible, e.g. a cube, a cuboid or a cone. The housing 2 may be made of a polymer which makes the housing 2 easy to manufacture. Different surface treatments of the housing 2 are envisaged, e.g. brushing, in order to give the housing 2 an elegant finish. The housing has a size of 29 cm x 9 cm x 8 cm. In general, the housing 2 should not be too large, e.g. below 30 cm x 10 cm x 10 cm or below 20 cm x 20 cm x 20 cm depending on its shape, such that the device 1 is easily portable and mobile.

The housing 2 comprises an inlet 21 and an outlet 22. In the device 1 of Fig. 1, the inlet 21 comprises a plurality of openings, e.g. with a diameter of less than 1 cm. The openings are distributed around the majority of the device' circumference in the lower part of the device. The "lower part" of the device 1 may be defined as the half of the device that is closer to a bottom support 14 of the device in an intended operating position. In operation, i.e. when the device 1 and in particular the fan in the device are switched on, air from the surroundings is sucked in through the inlet 21, i.e. through the openings. The location of the openings in the lower part of the device 1 supports an intake of larger and heavier airborne particles, which sink downwards, i.e. in the direction of gravity.

The outlet 22, on the other hand, is located in the upper part of the device 1, i.e. opposite to the inlet. Thus the air purified by the device is distributed over a larger area. In Fig. 1, the outlet 22 is formed by a ring slit in the top side 15 of the device 1, wherein the top side 15 is situated opposite to the bottom support 14.

Both, the air taken in through the inlet 21 and the air exhausted through the outlet 22, are schematically depicted as thick arrows in Fig. 1. The air path through the housing 2 in between inlet 21 and outlet 22 is not visible in Fig. 1 but shown in Fig. 2, see description further down.

Fig. 1 shows several further advantageous features of the mobile device 1. Embedded in the housing 2, there is a USB port 7 for supplying electrical power to the device. In general, other types of port or plug are possible for the power supply. The USB port 7, however, has the advantage that it can also be used for data transmission, e.g. an update of purification modes as input to the device or data from an air quality sensor of the device as output of the device. The USB port 7 may further be configured to provide electrical power, e.g. to a mobile device such as a smartphone.

Further, the device 1 comprises an input unit 12, which is advantageously located on the top side 15 of the housing 2 as depicted in Fig. 1 or on a side wall. The input unit 12 may comprise a gesture sensor which is configured to sense gestures of a user in the vicinity, i.e. up to 1 m distance, of the device 1. The input unit 12, in particular in connection with a control unit connected to it may categorize gestures of the user and use the gestures in order to control various parameters of the device. For instance, the user may control a speed of the fan, an intensity of the UV-C radiation source or a rate of negative ions generated by the source of negative ions via gestures such as waving.

In the embodiment of Fig. 1, the device 1 comprises a visual output unit 11 with a digital display and a color display. The digital display may e.g. be used for displaying a mode of operation or certain operating parameters of the device. The color display may comprise light-emitting diodes (LEDs) of different colors or a multicolor LED. The color display may be used to display a color-coded output, e.g. dependent on an operation mode of the device or on a sensed value of an air quality sensor of the device. In Fig. 1, the color display is arranged in the ring slit serving as outlet 22.

Fig. 2 shows a block diagram with functional elements of the device 1. Thick arrows again depict the air flow, here along the air path 23 in the housing 2 between the inlet 21 and the outlet 22. Thin lines depict electrical connections or other control connections, e.g. between control unit 13 and each of fan 3, UV-C radiation source 4 and source of ions 5.

It is to be understood that not all of the functional elements of Fig. 2 need to be present in a mobile air purifying device according to the invention. Further, the functional elements are not necessarily arranged in the order of Fig. 2 along the air path 23 in other embodiment of the invention.

In the device 1 of Fig. 2, the following functional elements are arranged in or, respectively, at the air path 23: the air quality sensor 10, a mechanical air filter 8, the fan 3, the UV-C radiation source 4, an odour source 9 and the source of negative ions 5.

The air quality sensor 10 may be a PM2.5 sensor and is advantageously arranged at the air path 23 near the inlet 21, in particular before any mechanical filter, e.g. before air filter 8. The air quality sensor 10 is connected to the visual output unit 11 described above. Advantageously, the air quality sensor 10 is also connected to a control unit 13 configured to process the values sensed by the air quality sensor 10.

The mechanical air filter 8 advantageously comprises a dust filter 81, an activated carbon filter 82 and a HEPA filter 83. In this way, the mechanical air filter 8 forms a cascade of ever-finer mesh size, with the dust filter 81 removing coarse dust particles from the air stream, the activated carbon filter 82 neutralizing gases and smells and the HEPA filter 83 removing fine particles, in particular with diameters below 0.1 µm. The mechanical air filter 8 is advantageously adapted to last for at least 500 hours of operation.

The fan 3 is powered by a battery 6 and controlled by the control unit 13. The plane of rotation of blades of the fan 3 may e.g. be oriented horizontally within the housing 2. The battery 6 is advantageously rechargeable, in particular via the USB port 7. Typically, the battery is a 12 V battery. The control unit 13 is connected to the input unit 12 for receiving user inputs as described above.

As a next stage, the UV-C radiation source 4 is arranged at the air path 23. In the mobile device 1, the UV-C radiation source 4 advantageously is a UV-C LED, which irradiates UV-C radiation on a part of the air path 23 within the housing 2. Advantageously, the UV-C radiation source 4 is shielded from all sides such that no UV-C radiation leaves the housing 2. Thus, a damage or injury of surrounding people through the UV-C radiation is prevented.

The odour source 9 may e.g. comprise a pad 91 which is adapted to receive scented oil, e.g. lavender oil. Such scented oil may e.g. be provided from a reservoir 92, which is advantageously located in the lower part of the housing 2. The pad 91 is further adapted to evaporate odorous substances form the scented oil into the air traveling along the air path 23. Moreover, the scented oil may have an anti-microbial and/or anti-viral effect.

Moreover, the source of negative ions 5 is arranged at the air path 23, advantageously near the outlet 22 and in particular after the mechanical air filter 8 and after the UV-C radiation source 4. The source of ions 5 generates ions, in particular negative ions, through ionization of air molecules. The negative ions then spread with the air flow over an area, e.g. of 10 m². Positively charged particles, in particular microorganisms or viruses, bind to the negatively charged ions and form clusters. Due to their higher weight, the clusters sink downwards, i.e. in direction of gravity, and accumulate near the floor. At the floor, the clusters may either be wiped away or be taken in by the inlet 21 of the device 1 again, where they are neutralized. In any case, the clusters are not inhaled by people in the surroundings.

It is understood that a device 1 with the above features is mobile and easily adjustable to various situations of every-day life, e.g. in an office, a meeting room, a car etc. The device effectively purifies air in its surroundings and thus improves air quality and the well-being of people. Further, the device prevents the transmission of diseases that spread via aerosols or droplets such as Covid-19.

## Claims

1. A mobile air purifying device (1), comprising
- a housing (2) with an inlet (21) and an outlet (22) for air,
- an air path (23) through the housing (2) between the inlet (21) and the outlet (22),
- a fan (3) adapted to draw air along the air path (23) through the housing (2),
- a UV-C radiation source (4) adapted to emit UV-C radiation onto a part of the air path (23),
- a source of ions (5) adapted to generate ions in the air path (23).

2. The device of claim 1, further comprising
- a battery (6) for supplying power to the fan (3), the UV-C radiation source (4) and the source of ions (5), independent from a location of the device,
in particular wherein the battery (6) is adapted to be recharged via a USB port (7) of the device, and
in particular wherein the battery (6) is adapted to supply power to an external device via the USB port (7).

3. The device of any of the preceding claims, further comprising
- a mechanical air filter (8) within the air path (23),
in particular wherein the mechanical air filter (8) comprises at least one of the following:
- a dust filter (81),
- an activated carbon filter (82),
- a HEPA filter (83), in particular of HEPA class H13 or higher purifying efficiency.

4. The device of any of the preceding claims, further comprising
- an odour source (9), in particular a pad (91) for receiving scented oil, adapted to odorize air drawn through the air path (23).

5. The device of any of the preceding claims, wherein the UV-C radiation source (4) comprises a UV-C LED.

6. The device of any of the preceding claims, wherein the source of ions (5) comprises a negative ion generator adapted to ionize air molecules, in particular at least 5 million, more particularly at least 8 million, air molecules per second.

7. The device of any of the preceding claims weighing less than 1 kg, in particular less than 0.5 kg.

8. The device of any of the preceding claims, wherein the housing (2) has a height of less than 30 cm, a width of less than 10 cm and a length of less than 10 cm.

9. The device of any of the preceding claims, further comprising
- an air quality sensor (10), in particular a PM2.5 sensor, adapted to sense a quality of air drawn into the inlet (21),
- a visual output unit (11) connected to the air quality sensor (10) and adapted to visually output an air quality value from the air quality sensor (10),
in particular wherein the visual output unit (11) comprises a multicolor LED or at least two LEDs of different colors.

10. The device of any of the preceding claims, further comprising
- an input unit (12), and
- a control unit (13) adapted to control the fan (3), the UV-C radiation source (4) and the source of ions (5) according to a user input to the input unit (12) .

11. The device of any of the preceding claims,
wherein the input unit (12) comprises a gesture sensor, in particular comprising an IR transmitter and receiver, adapted to contactlessly sense a user input.

12. The device of any of the preceding claims, further comprising
- a bottom support (14) for supporting the housing (2) in regular operation,
wherein the inlet (21) is arranged closer to the bottom support (14) than the outlet (22),
in particular wherein the inlet (21) is arranged in a side wall of the housing (2) adjacent to the bottom support (14), and/or
in particular wherein the outlet (22) is arranged in an upper wall (15) of the housing (2) opposite to the bottom support (14).

13. Method for purifying air by means of a mobile air purifying device (1) according to any of the preceding claims, comprising
- exhausting negative ions generated by the ion source (5) through the outlet (22) into the ambient air for the negative ions electrostatically binding to one or more of particles, microorganisms or viruses in the ambient air, resulting in a cluster sinking to ground, and
- drawing the cluster via the inlet (21) into the air path (23) of the device (1), and
- filtering the cluster by means of the mechanical air filter, in particular by means of the HEPA filter, or
- in case the cluster passing the mechanical air filter, irradiating the cluster by UV-C radiation emitted by the UV-C radiation source (4) for destroying or inactivating any of the particles, microorganisms or viruses bound in the cluster.
